# EUROPEAN PATENT APPLICATION

(11) **EP 0 612 528 A1**
(43) Date of publication of application: **31.08.1994**
(21) Application number: 94102553.8
(22) Date of filing: 21.02.1994
(51) Int. Cl.: A61K 33/14, A61M 1/28

(54) **Solutions for peritoneal dialysis**

(30) Priority: 24.02.1993 IT MI930360
(71) Applicant: B. BRAUN CAREX S.p.A., I-41037 Mirandola (Modena) (IT)
(72) Inventor: Bellini, Gianni, I-41036 Medolla (Modena) (IT); Gavioli, Giuliana, I-41037 Mirandola (Modena) (IT); Von der Haar, Friedrich, 34212 Melsungen (DE)
(74) Representative: Modiano, Guido, Dr.-Ing.

(57) **Abstract**

A peritoneal dialysis solution that comprises electrolytes that contain ions chosen among ions of sodium, potassium, calcium, magnesium and chlorine, characterized in that it comprises an osmotic substance chosen among gluconic acid and its pharmaceutically acceptable salts.

## Description

The present invention relates to solutions for peritoneal dialysis that contain an osmotic substance which is an alternative to glucose.

Peritoneal dialysis is a treatment for acute or chronic renal failure that is considered on an equal level with hemodialysis and kidney transplantation. Recent studies have shown that the survival of patients treated continuously by peritoneal dialysis can be compared with that of patients treated by hemodialysis, that complications involving peritonitis are significantly reduced, and that alterations of the peritoneal membrane are scarcely relevant when complications involving peritonitis are prevented and when dialysis solutions that are as physiological as possible are used.

Peritoneal dialysis solutions are similar to plasma in their electrolytic composition and are sterile solutions which are checked for the limit of bacterial endotoxins. The quality of peritoneal dialysis solutions has been improved considerably thanks to the increasing attention paid to the evaluation of the raw materials used and of the production processes.

Peritoneal dialysis solutions have variable compositions. The main components of these solutions include ions of sodium, potassium, magnesium, acetate or lactate, chlorides and glucose in a variable percentage which is in any case comprised between 1% and 5%. Glucose is the largest osmotic component of the solution.

Known formulae for peritoneal dialysis may or may not contain bicarbonate. However, the formulae that contain bicarbonate are less preferred, since their components (potassium and calcium salts) precipitate at the basic pH of a bicarbonate-containing solution when they are mixed together.

Known peritoneal dialysis solutions contain variable concentrations of osmotically active substances. Currently, the only osmotic substance used to induce ultrafiltration in peritoneal dialysis is glucose. In practice, solutions that have an osmolarity of 200 to 600 mOsm/l are used. Theoretical osmolarity is given by the sum of the mmols of the various components (ions and glucose).

Although glucose is effective in subtracting liquids, its use in peritoneal dialysis solutions is not free from drawbacks. The high concentrations in glucose and degradation products thereof in fact have negative effects on the peritoneal membrane. Besides, the high reabsorption of glucose from the dialysis solution alters glycolipid metabolism, can cause obesity and facilitate hypertriglyceridemia, glucose intolerance, and alterations in insulin and glucagon hormone levels, and this creates particular problems in patients with diabetes. In addition, glucose has a relatively short-lasting osmotic effect and requires acidification of the solution to avoid crystallization during sterilization; this acidification has negative effects on the defensive capabilities of neutrophils in producing oxygen radicals that have a bactericidal activity in the peritoneal cavity.

In vivo and in vitro physiopathological studies of the peritoneal membrane have provided much useful information on the use of more appropriate solutions and on the long-term vitality of the membrane.

In order to reduce the use of glucose in peritoneal dialysis solutions, it has been proposed to use alternative osmotic agents such as sorbitol, mannitol, fructose, glycerol, dextrose, xylitol, peptide mixtures and gelatin. However, these agents either do not have actual advantages over glucose or cannot be used because they have a toxic effect.

A principal aim of the present invention is to eliminate the drawbacks described above in known types of peritoneal dialysis solutions, providing a peritoneal dialysis solution that does not harm the physical and physiological properties of the peritoneum.

Another aim of the present invention is to provide a peritoneal dialysis solution which is non-toxic and well-tolerated.

Another aim of the present invention is to provide a peritoneal dialysis solution that is not harmful from a biochemical and metabolic point of view.

With these aims and other objects of the invention which will become apparent hereinafter, in view, there is provided a peritoneal dialysis solution according to the invention, characterized in that it comprises an osmotic substance chosen among gluconic acid and its pharmaceutically acceptable salts.

Preferably, the peritoneal dialysis solution according to the present invention also comprises lactate or, as a replacement thereof, bicarbonate.

Advantageously, gluconic acid and/or any salts thereof are included in the peritoneal dialysis solution according to the present invention at a concentration of 1 to 5% by weight and therefore of 10 g/l up to 50 g/l.

Conveniently, the total concentration of gluconic acid and salts thereof in the peritoneal dialysis solution according to the present invention is such that the final osmolarity of the solution is between 200 and 600 mOsm/l. This osmolarity can be achieved also by virtue of the simultaneous presence of gluconic acid and/or salts thereof and glucose. It is possible to use mixtures of gluconic acid or salts thereof and glucose, in which the gluconic acid or salts thereof are present in an amount comprised between 10 and 90%.

The gluconic acid salts that can be contained in the peritoneal dialysis solution according to the present invention are preferably chosen among the group formed by calcium gluconate, zinc gluconate, sodium gluconate, sodium stibogluconate, magnesium gluconate and iron gluconate.

The concentration range of the sodium, potassium, calcium, magnesium and chlorine ions in the solution is the one that is used normally, for example the one specified in the Italian "Formulario Nazionale" for peritoneal dialysis solutions, i.e. 125 to 145 mEq for the sodium ion, 0 to 4.0 mEq for the potassium ion, 2.0 to 5.0 mEq for the calcium ion, 0.5 to 1.5 mEq for the magnesium ion; 90 to 120 mEq for the chlorine ion (such that the concentration of chlorine ions is such as to balance the total ion concentration); and 30-45 Eq/l for the buffer (acetate-lactate-bicarbonate).

The peritoneal dialysis solutions according to the present invention may also comprise a stabilizing agent.

The following examples illustrate the invention by way of non-limitative example.

### EXAMPLE 1

A solution that contains gluconic acid and/or salts thereof or a mixture of glucose or gluconic acid and/or salts thereof in a single container.
CONTAINER: plastic sac (medical-grade PVC or other medical-grade material)
PRODUCTION: filling-packaging in an overpouch-steam sterilization.
FORMULA: 125 to 145 mEq Na⁺, 0 to 4 mEq K⁺, 2.00 to 5.00 mEq Ca⁺⁺, 0.5 to 1.5 mEq Mg⁺⁺, 35 to 45 mEq acetate or lactate, 90 to 120 mEq Cl⁻, 75 to 250 mmol gluconic acid and/or salts thereof (or 75 to 250 mmol gluconic acid and/or salts thereof and glucose).

The concentration of gluconic acid and/or salts thereof in the mixture of glucose and gluconic acid and/or salts thereof can be 10 to 90%.

### EXAMPLE 2

A CAPD solution containing gluconic acid and/or salts thereof (or a mixture of gluconic acid and/or salts thereof and glucose) and sodium bicarbonate.
CONTAINER: two-chamber sac or double sac made of medical-grade plastic.
PRODUCTION: filling-packaging in an overpouch - steam sterilization.

The final solution is the result of the mixing, performed just before use, of two solutions: an acid one with gluconic acid and/or salts thereof or gluconic acid and/or salts thereof and glucose, and a basic sodium bicarbonate solution.

The final solution has the following concentration:
2 to 145 mEq Na⁺, 0 to 4 mEq K⁺, 2 to 5 mEq Ca⁺⁺, 0.5 to 1.5 mEq Mg⁺⁺, 90 to 120 mEq Cl⁻, 75 to 250 mmol gluconic acid and/or salts thereof mixed or not with glucose, 30 to 45 mEq HCO3⁻ (bicarbonate).

The basic solution has the following composition:
a solution containing sodium bicarbonate (NaHCO₃) at such a concentration as to give the final solution a bicarbonate concentration of 30 to 45 mEq. For example, if the basic solution is mixed with the acid solution in equal parts (1:1), the concentration of the bicarbonate basic solution will be 60 to 90 mEq.

The basic solution has a pH comprised between 7 and 8.5.

Acid solution: a solution that contains all the other electrolytes at such a concentration as to obtain, after mixing with the basic solution, a concentration that lies within the following ranges:
2 to 145 mEq Na⁺, 0 to 4 mEq K⁺, 2 to 5 mEq Ca⁺⁺, 0.5 to 1.5 mEq Mg⁺⁺, 90 to 120 mEq Cl⁻, 75 to 250 mmol gluconic acid and/or salts thereof (75 to 250 mmol gluconic acid and/or salts thereof and glucose).

If, as in the preceding example, the mixture is 1:1, the concentration of the acid solution will be doubled for each electrolyte and specifically: 4 to 290 Na⁺, etc.

The basic solution is contained in a separate sac (made of medical-grade PVC or other medical-grade plastic) equipped with a membrane connector. This connector is shaped complementarily to a connector provided with a perforator that is assembled on the sac that contains the acid solution, which is constituted by the electrolytes and the gluconic acid and/or salts thereof or a mixture of gluconic acid and/or salts thereof and glucose.

The two solutions, the acid one and the basic one, can also be contained in a two-chamber sac and mixed by means of a break-open connector or of a peel-open weld inside the sac.

Thus, if bicarbonate is used, the final dialysis solution can be obtained by mixing two solutions, one of which is basic, contained in two different containers or in a two-chamber container to avoid the precipitation of salts (calcium/potassium) or of the bicarbonate itself. The solution can be provided in the form of a concentrated solution to be diluted at the time of use.

In vitro tests on cultures of peritoneal mesothelial cells have shown that gluconic acid and its salts do not have nutrient characteristics (and therefore do not allow the growth of cells in cultures) but yield good results in peritoneal-mesothelial cell confluence cultures, which means that the gluconic acid and/or salts thereof give an osmotic power to the solution without the negative effects of glucose.

In particular, the study conducted by the Applicant shows that gluconic acid and its salts are non-toxic and well-tolerated, do not harm the physical and physiological properties of the peritoneum and are not harmful from a biochemical and metabolic point of view. However, they obviate all the drawbacks mentioned with reference to glucose, particularly for the treatment of patients with diabetes.

## Claims

1. Peritoneal dialysis solution, comprising electrolytes that contain ions chosen among ions of sodium, potassium, calcium, magnesium, chlorine and mixtures thereof, characterized in that it comprises at least one osmotic substance chosen among gluconic acid and its pharmaceutically acceptable salts.

2. Peritoneal dialysis solution according to claim 1, characterized in that it also comprises lactate.

3. Peritoneal dialysis solution according to claim 1, characterized in that it also comprises bicarbonate.

4. Peritoneal dialysis solution according to any one of the preceding claims, wherein the gluconic acid and/or its salts are included at a concentration of 1 to 5% by weight.

5. Peritoneal dialysis solution according to any one of the preceding claims, which comprises a mixture of gluconic acid or salts thereof with glucose as osmotic substances.

6. Peritoneal dialysis solution according to any one of the preceding claims, characterized in that it has a total osmolarity of 220 to 600 mOsm/l.

7. Peritoneal dialysis solution according to any one of the preceding claims, characterized in that said gluconic acid salts are chosen in the group formed by calcium gluconate, zinc gluconate, sodium gluconate, sodium stibogluconate, magnesium gluconate and iron gluconate.

8. Peritoneal dialysis solution according to any one of the preceding claims, characterized in that it has a concentration of 125 to 145 mEq for the sodium ion, 0 to 4.0 mEq for the potassium ion, 2.0 to 5.0 mEq for the calcium ion, 0.5 to 1.5 mEq for the magnesium ion, and 90 to 120 mEq for the chlorine ion.

9. Peritoneal dialysis solution according to any one of the preceding claims, characterized in that it also comprises a stabilizing agent.

10. Kit for peritoneal dialysis, comprising at least one sealed container that contains a dialysis solution according to any one of the preceding claims.

11. Kit according to claim 10, comprising two containers, a first one of said containers comprising a basic bicarbonate solution and a second one of said containers comprising the remaining part of the dialysis solution according to claim 3, said containers being suitable to be connected to mix their contents.

12. Kit according to claim 10, comprising a two-chamber container, one first chamber containing a bicarbonate solution, the second chamber containing the remaining part of the dialysis solution according to claim 3, said chambers being suitable to be connected in order to mix their contents.
